# EUROPÄISCHE PATENTSCHRIFT

(11) **EP 3 192 146 B1**
(45) Veröffentlichungstag und Bekanntmachung des Hinweises auf die Patenterteilung: **23.10.2019**
(21) Anmeldenummer: 15735910.0
(22) Anmeldetag: 02.07.2015
(51) Int. Cl.: H02J 50/00, H02J 7/02, A41D 1/00

(54) **AUFBEWAHRUNGSSYSTEM**
STORAGE SYSTEM
SYSTÈME D'ENTREPOSAGE

(30) Priorität: 09.09.2014 DE 102014217965
(43) Veröffentlichungstag der Anmeldung: 19.07.2017
(73) Patentinhaber: Robert Bosch GmbH, 70442 Stuttgart (DE)
(72) Erfinder: SCHADOW, Joachim, 70563 Stuttgart (DE); LUTZ, Manfred, 70794 Filderstadt (DE); STOCK, Joern, 72658 Bempflingen (DE); ESENWEIN, Florian, 70771 Leinfelden-Echterdingen (DE); BARTH, Daniel, 70771 Leinfelden-Echterdingen (DE); BOECK, Cornelius, 73230 Kirchheim (DE); MAUTE, Joerg, 71069 Sindelfingen (DE)
(86) Internationale Anmeldenummer: PCT/EP2015/065141
(87) Internationale Veröffentlichungsnummer: WO 2016/037722

(56) Entgegenhaltungen:
- EP-A2- 2 365 698
- WO-A1-2010/093723
- JP-A- 2004 033 596
- US-A1- 2006 087 282
- US-A1- 2014 015 336

## Beschreibung

### Stand der Technik

Aus DE 20 2007 004 441 U1 ist bereits ein Aufbewahrungssystem bekannt, das eine Aufbewahrungsvorrichtung zu einer Aufbewahrung von Arbeitskleidung, eine kabellose Ladevorrichtung, die dazu vorgesehen ist, elektrische Energie an eine Energiespeichereinheit der Arbeitskleidung zu übertragen, und das eine Sensorvorrichtung zu einer Erfassung einer Kenngröße der Arbeitskleidung umfasst. Mittels der Ladevorrichtung des bekannten Aufbewahrungssystems ist hierbei ein spezielles Arbeitskleidungsteil aufladbar.

Aus der US 2014/015336 A1 ist weiterhin ein Aufbewahrungssystem gemäß dem Oberbegriff des Anspruchs 1 bekannt.

### Offenbarung der Erfindung

Erfindungsgemäss wird ein Aufbewahrungssystem gemäß Anspruch 1 bereitgestellt.

Die Erfindung geht aus von einem Aufbewahrungssystem, insbesondere von einem Arbeitskleidungsaufbewahrungssystem, mit zumindest einer Aufbewahrungsvorrichtung zumindest zu einer Aufbewahrung von Arbeitskleidung, mit zumindest einer kabelgebundenen und/oder kabellosen Ladevorrichtung, die dazu vorgesehen ist, zumindest elektrische Energie an zumindest eine Energiespeichereinheit der Arbeitskleidung zu übertragen, insbesondere in zumindest einem an der Aufbewahrungsvorrichtung angeordneten Zustand der Arbeitskleidung, und mit zumindest einer Sensorvorrichtung zumindest zu einer Erfassung von zumindest einer Kenngröße der Arbeitskleidung.

Es wird vorgeschlagen, dass die Ladevorrichtung dazu vorgesehen ist, verschiedene Arbeitskleidungsteile der Arbeitskleidung, die an der Aufbewahrungsvorrichtung anordenbar sind, zu laden. Besonders bevorzugt ist das Aufbewahrungssystem als Arbeitskleidungsspindsystem ausgebildet. Es ist jedoch auch denkbar, dass das Aufbewahrungssystem eine andere, einem Fachmann als sinnvoll erscheinende Ausgestaltung aufweist, wie beispielsweise eine Ausgestaltung als Kleiderschranksystem, als Kommodensystem, als Werkstattwagensystem, als Regalsystem, als Fahrzeuginnenraumausbauaufbewahrungssystem o. dgl. Somit ist die Aufbewahrungsvorrichtung bevorzugt als Spind ausgebildet. Es ist jedoch auch denkbar, dass die Aufbewahrungsvorrichtung eine andere, einem Fachmann als sinnvoll erscheinende Ausgestaltung aufweist. Besonders bevorzugt umfasst das Aufbewahrungssystem eine Vielzahl an Aufbewahrungsvorrichtungen. Insbesondere sind die Aufbewahrungsvorrichtungen miteinander verbunden. Vorzugsweise ist die Aufbewahrungsvorrichtung verschließbar ausgebildet. Hierbei ist vorzugsweise ein Aufbewahrungsraum der Aufbewahrungsvorrichtung verschließbar. Somit umfasst die Aufbewahrungsvorrichtung bevorzugt zumindest eine Verschlusseinheit, die zumindest ein beweglich gelagertes Verschlusselement umfasst. Insbesondere ist die Verschlusseinheit als Türeinheit ausgebildet. Es ist jedoch auch denkbar, dass die Verschlusseinheit eine andere, einem Fachmann als sinnvoll erscheinende Ausgestaltung aufweist. Das Verschlusselement ist vorzugsweise beweglich, insbesondere schwenkbar, an einem Korpus der Aufbewahrungsvorrichtung gelagert. Somit kann vorteilhaft mittels der Verschlusseinheit ein Innenraum der Aufbewahrungsvorrichtung, in dem zumindest eine Aufbewahrungseinheit der Aufbewahrungsvorrichtung anordenbar ist, geschlossen werden.

Vorteilhafterweise umfasst die Aufbewahrungsvorrichtung zu einer Aufbewahrung von Arbeitskleidung zumindest eine als Kleiderstange, eine als Kleiderbügel, eine als Kleiderhaken, eine als Regalboden, eine als Schublade, eine als Staufach und/oder eine als weitere, einem Fachmann als sinnvoll erscheinende Aufbewahrungseinheit auf, die dazu vorgesehen ist, Arbeitskleidung oder Arbeitskleidungsteile aufzunehmen. Die Aufbewahrungsvorrichtung ist bevorzugt zusätzlich zu einer Aufnahme von Arbeitskleidung zu einer Aufnahme von zumindest einer Werkzeugmaschinenakkumulatorvorrichtung, von zumindest einer externen Einheit, insbesondere einem tragbaren Mobilfunkgerät, wie beispielswiese einem Handy, einem Smartphone, einem Tablet usw., von zumindest einer tragbaren Werkzeugmaschine o. dgl. vorgesehen. Unter "vorgesehen" soll insbesondere speziell ausgelegt und/oder speziell ausgestattet verstanden werden. Darunter, dass ein Element und/oder eine Einheit zu einer bestimmten Funktion vorgesehen ist, soll insbesondere verstanden werden, dass das Element und/oder die Einheit diese bestimmte Funktion in zumindest einem Anwendungs- und/oder Betriebszustand erfüllen/erfüllt und/oder ausführen/ausführt.

Unter einer "kabelgebundenen Ladevorrichtung" soll hier insbesondere eine Ladevorrichtung verstanden werden, die elektrische Energie mittels zumindest eines Ladekabels an ein zu ladendes Element und/oder an eine zu ladende Einheit überträgt. Der Begriff "kabellose Ladevorrichtung" soll hier insbesondere eine Ladevorrichtung definieren, die elektrische Energie kontaktlos an ein zu ladendes Element und/oder an eine zu ladende Einheit überträgt, insbesondere mittels einer induktiven, einer kapazitiven oder einer elektromagnetischen Energieübertragung. Hierbei ist es denkbar, dass die Ladevorrichtung zumindest eine Induktivladeeinheit umfasst und zusätzlich eine Kabelladeeinheit. Somit könnte in Abhängigkeit einer Ausgestaltung eines Ladegegenstücks der Arbeitskleidung zuverlässig eine Lademöglichkeit für unterschiedlich ausgestaltete Ladegegenstücke der Arbeitskleidung ermöglicht werden.

Bevorzugt ist die Ladevorrichtung als Induktivladevorrichtung ausgebildet. Somit umfasst die Ladevorrichtung vorzugsweise zumindest eine Sekundärspuleneinheit zu einer Übertragung einer elektrischen Energie, wobei die Arbeitskleidung, insbesondere alle Arbeitskleidungsteile der Arbeitskleidung, zumindest eine Primärspuleneinheit zu einem Empfang der elektrischen Energie aufweist, insbesondere aufweisen. Hierbei sind vorzugsweise unterschiedliche Nennspannungen zu einer Übertragung von elektrischer Energie nutzbar. Die Arbeitskleidung, insbesondere alle Arbeitskleidungsteile der Arbeitskleidung, umfasst bzw. umfassen bevorzugt zumindest eine Energiespeichereinheit, die als Akkumulator ausgebildet ist. Hierbei weist die Energiespeichereinheit vorzugsweise eine Nennspannung von 3,6 V auf. Es ist jedoch auch denkbar, dass die Energiespeichereinheit der Arbeitskleidung eine andere, einem Fachmann als sinnvoll erscheinende Ausgestaltung und/oder Nennspannung aufweist. Bevorzugt ist die Ladevorrichtung zusätzlich zu einer Übertragung von elektrischer Energie an die Arbeitskleidung zu einer Übertragung von elektrischer Energie an zumindest eine Akkumulatorvorrichtung, an zumindest eine externe Einheit, insbesondere ein tragbares Mobilfunkgerät, wie beispielsweise ein Handy, ein Smartphone, ein Tablet usw., an zumindest eine tragbare Werkzeugmaschine o. dgl. vorgesehen, insbesondere in einem an der Aufbewahrungsvorrichtung und/oder an der Ladevorrichtung angeordneten Zustand. Ferner ist die Ladevorrichtung bevorzugt in die Aufbewahrungsvorrichtung integriert.

Die Sensorvorrichtung ist vorzugsweise zu einer Erfassung von zumindest einer Ladezustandskenngröße der Energiespeichereinheit der Arbeitskleidung, insbesondere der Energiespeichereinheit des jeweiligen Arbeitskleidungsteils der Arbeitskleidung, vorgesehen. Zudem ist es denkbar, dass die Sensorvorrichtung zu einer Erfassung von weiteren, einem Fachmann als sinnvoll erscheinenden Kenngrößen der Arbeitskleidung vorgesehen ist, wie beispielsweise zu einer Erfassung von einer korrekten Anordnung der Arbeitskleidung an der Aufbewahrungsvorrichtung und/oder an der Ladevorrichtung o. dgl.

Darunter, dass "die Ladevorrichtung dazu vorgesehen ist, verschiedene Arbeitskleidungsteile der Arbeitskleidung, die an der Aufbewahrungsvorrichtung anordenbar sind, zu laden" soll hier insbesondere verstanden werden, dass die Ladevorrichtung derart ausgebildet ist, dass zumindest elektrische Energie an verschiedene Bestandteile der Arbeitskleidung mittels einer einzelnen Ladevorrichtung übertragbar ist, wie beispielsweise an Arbeitshandschuhe, an einen Schutzhelm, an Handschuhe, an eine Arbeitshose, an eine Schutzbrille, an einen Gürtel und/oder an andere, einem Fachmann als sinnvoll erscheinende Arbeitskleidungsteile der Arbeitskleidung. Hierbei ist es denkbar, dass alle Arbeitskleidungsteile der Arbeitskleidung gleichzeitig mit der Ladevorrichtung aufladbar sind, insbesondere in einem an der Aufbewahrungsvorrichtung angeordneten Zustand der verschiedenen Arbeitskleidungsteile der Arbeitskleidung. Mittels der erfindungsgemäßen Ausgestaltung der Erfindung kann vorteilhaft ein Aufbewahrungssystem zur Verfügung gestellt werden, das eine komfortable Lademöglichkeit von verschiedenen Arbeitskleidungsteilen der Arbeitskleidung ermöglicht. Somit kann vorteilhaft ein zentrales Aufbewahrungssystem realisiert werden, das zu einem Laden von einzelnen Energiespeichereinheiten von verschiedenen Arbeitskleidungsteilen der Arbeitskleidung nutzbar ist. Es kann vorteilhaft ein komfortabel zu bedienendes und zu bestückendes Aufbewahrungssystem realisiert werden. Ein Träger von Arbeitskleidung kann somit vorteilhaft seine gesamte Arbeitskleidung mit einem einzelnen Aufbewahrungssystem mit elektrischer Energie versorgen, insbesondere in einem an der Aufbewahrungsvorrichtung angeordneten Zustand der verschiedenen Arbeitskleidungsteile der Arbeitskleidung.

Des Weiteren wird vorgeschlagen, dass die Aufbewahrungsvorrichtung zumindest eine Schutzhelmaufbewahrungseinheit umfasst, in der zumindest ein als Schutzhelm ausgebildetes Arbeitskleidungsteil der Arbeitskleidung anordenbar ist, wobei zumindest eine Schutzhelmladeeinheit der Ladevorrichtung zu einem Laden zumindest einer Energiespeichereinheit des Schutzhelms an der Schutzhelmaufbewahrungseinheit angeordnet ist, insbesondere in die Schutzhelmaufbewahrungseinheit integriert ist. Die Schutzhelmladeeinheit ist vorzugsweise als kontaktlose Schutzhelmladeeinheit ausgebildet, insbesondere als Induktivschutzhelmladeeinheit. Mittels der erfindungsgemäßen Ausgestaltung kann vorteilhaft ein als Schutzhelm ausgebildetes Arbeitskleidungsteil der Arbeitskleidung komfortabel aufbewahrt werden und geladen werden.

Ferner wird vorgeschlagen, dass die Aufbewahrungsvorrichtung zumindest eine Handschuhaufbewahrungseinheit umfasst, in der zumindest ein als Handschuhpaar ausgebildetes Arbeitskleidungsteil der Arbeitskleidung anordenbar ist, wobei zumindest eine Handschuhladeeinheit der Ladevorrichtung zu einem Laden zumindest einer Energiespeichereinheit des Handschuhpaars an der Handschuhaufbewahrungseinheit angeordnet ist, insbesondere in die Handschuhaufbewahrungseinheit integriert ist. Die Handschuhladeeinheit ist vorzugsweise als kontaktlose Handschuhladeeinheit ausgebildet, insbesondere als Induktivhandschuhladeeinheit. Mittels der erfindungsgemäßen Ausgestaltung kann vorteilhaft ein als Handschuhpaar ausgebildetes Arbeitskleidungsteil der Arbeitskleidung komfortabel aufbewahrt werden und geladen werden.

Zudem wird vorgeschlagen, dass die Aufbewahrungsvorrichtung zumindest eine Arbeitsschuhaufbewahrungseinheit umfasst, in der zumindest ein als Arbeitsschuhpaar ausgebildetes Arbeitskleidungsteil der Arbeitskleidung anordenbar ist, wobei zumindest eine Arbeitsschuhladeeinheit der Ladevorrichtung zu einem Laden zumindest einer Energiespeichereinheit des Arbeitsschuhpaars an der Arbeitsschuhaufbewahrungseinheit angeordnet ist, insbesondere in die Arbeitsschuhaufbewahrungseinheit integriert ist. Die Arbeitsschuhladeeinheit ist vorzugsweise als kontaktlose Arbeitsschuhladeeinheit ausgebildet, insbesondere als Induktivarbeitsschuhladeeinheit. Ferner ist es denkbar, dass die Aufnahmevorrichtung weitere, einem Fachmann als sinnvoll erscheinende Aufbewahrungseinheiten umfasst, wie beispielsweise eine Arbeitsgürtelaufbewahrungseinheit, eine Arbeitssockenaufbewahrungseinheit, eine Arbeitsunterwäscheaufbewahrungseinheit, wobei an den jeweiligen weiteren Aufbewahrungseinheiten jeweils eine Ladeeinheit der Ladevorrichtung zu einem Laden einer Energiespeichereinheit eines entsprechenden Arbeitskleidungsteils angeordnet ist. Mittels der erfindungsgemäßen Ausgestaltung kann vorteilhaft ein als Arbeitsschuhpaar ausgebildetes Arbeitskleidungsteil der Arbeitskleidung komfortabel aufbewahrt werden und geladen werden.

Des Weiteren wird vorgeschlagen, dass die Aufbewahrungsvorrichtung zumindest eine Schutzbrillenaufbewahrungseinheit umfasst, in der zumindest eine als Schutzbrille ausgebildetes Arbeitskleidungsteil der Arbeitskleidung anordenbar ist, wobei zumindest eine Schutzbrillenladeeinheit der Ladevorrichtung zu einer Energieversorgung zumindest einer Energiespeichereinheit der Schutzbrille an der Schutzbrillenaufbewahrungseinheit angeordnet ist, insbesondere in die Schutzbrillenaufbewahrungseinheit integriert ist. Die Schutzbrillenladeeinheit ist vorzugsweise als kontaktlose Schutzbrillenladeeinheit ausgebildet, insbesondere als Induktivschutzbrillenladeeinheit. Mittels der erfindungsgemäßen Ausgestaltung kann vorteilhaft ein als Schutzbrille ausgebildetes Arbeitskleidungsteil der Arbeitskleidung komfortabel aufbewahrt werden und geladen werden.

Vorteilhafterweise wird vorgeschlagen, dass die Ladevorrichtung dazu vorgesehen ist, zumindest eine Werkzeugmaschinenakkumulatorvorrichtung und/oder zumindest eine Energiespeichereinheit einer externen Einheit zu laden, insbesondere in einem an der Aufbewahrungsvorrichtung angeordneten Zustand der Werkzeugmaschinenakkumulatorvorrichtung und/oder der externen Einheit. Vorzugsweise umfasst die Ladevorrichtung hierzu zumindest eine Werkzeugmaschinenakkumulatorladeeinheit und/oder eine Ladeeinheit zu einem Laden der externen Einheit. Zudem umfasst die Aufbewahrungsvorrichtung vorzugsweise zumindest eine Werkzeugmaschinenaufbewahrungseinheit und/oder eine Aufbewahrungseinheit zu einem Aufbewahren der externen Einheit. Vorzugsweise sind/ist die Werkzeugmaschinenakkumulatorladeeinheit und/oder die Ladeeinheit zu einem Laden der externen Einheit in die Werkzeugmaschinenaufbewahrungseinheit und/oder in die Aufbewahrungseinheit zu einem Aufbewahren der externen Einheit integriert. Die externe Einheit ist vorzugsweise als tragbares Mobilfunkgerät ausgebildet, wie beispielsweise ein Handy, ein Smartphone, ein Tablet o. dgl. Es ist jedoch auch denkbar, dass die externe Einheit eine andere, einem Fachmann als sinnvoll erscheinende Ausgestaltung aufweist. Mittels der erfindungsgemäßen Ausgestaltung kann vorteilhaft ein breites Einsatzspektrum des Aufbewahrungssystems realisiert werden.

Ferner wird vorgeschlagen, dass das Aufbewahrungssystem zumindest eine Kommunikationsvorrichtung zumindest zu einem Austausch von elektronischen Daten mit der Arbeitskleidung, insbesondere in zumindest einem an der Aufbewahrungsvorrichtung angeordneten Zustand der Arbeitskleidung, umfasst. Die Kommunikationsvorrichtung ist vorzugsweise als kabellose Kommunikationsvorrichtung ausgebildet. Hierbei kann die Kommunikationsvorrichtung als WLAN-Kommunikationsvorrichtung, als Bluetooth-Kommunikationsvorrichtung, als Funk-Kommunikationsvorrichtung, als RFID-Kommunikationsvorrichtung, als NFC-Vorrichtung, als Infrarot-Kommunikationsvorrichtung, als Mobilfunknetz-Kommunikationsvorrichtung, als Zigbee-Kommunikationsvorrichtung o. dgl. ausgebildet sein. Besonders bevorzugt ist die Kommunikationsvorrichtung zu einer bidirektionalen Datenübertragung vorgesehen. In einer alternativen Ausgestaltung ist die Kommunikationsvorrichtung als kabelgebundene Kommunikationsvorrichtung ausgebildet, wie beispielsweise als LAN-Kommunikationsvorrichtung, als USB-Kommunikationsvorrichtung, als Powerline-Kommunikationsvorrichtung, als Canbus-Kommunikationsvorrichtung, als Ethernet-Kommunikationsvorrichtung, als twisted pair Kabel-Kommunikationsvorrichtung (CAT5 oder CAT6) o. dgl. Es ist jedoch auch denkbar, dass die Kommunikationsvorrichtung alternativ zu einer kabellosen oder zu einer kabelgebundenen Kommunikation zu einer kabellosen und zu einer kabelgebunden Kommunikation vorgesehen ist. Mittels der erfindungsgemäßen Ausgestaltung kann vorteilhaft ein Austausch von elektronischen Daten zu einer Überwachung der Arbeitskleidung ermöglicht werden.

Erfindungsgemäss sind mittels der Kommunikationsvorrichtung elektronische Daten zwischen der Arbeitskleidung und einer externen Einheit austauschbar. Die externe Einheit kann als Smartphone, als PersonalComputer, als Laptop, als Netbook, als Tablet, als Firmenzentralrechner, als tragbare Werkzeugmaschine, als Ausgabeeinheit, wie beispielsweise als Lautsprecher oder als eine andere, einem Fachmann als sinnvoll erscheinende externe Einheit ausgebildet sein. Bei einer Ausgestaltung als Smartphone, als Personal-Computer, als Laptop, als Netbook oder als Tablet ist vorzugsweise eine App zu einer Kommunikation mit der Kommunikationsvorrichtung vorgesehen. Es ist jedoch auch denkbar, dass die externe Einheit als externe, transportable Bedieneinheit, als fest installierte Bedieneinheit an einem Arbeitsplatz eines Bedieners, als fest in einem Raum installierte Synchronisationseinheit eines Einsatzortes, die von einer Zentrale gesteuert werden kann, wie beispielsweise infolge von Firmenvorgaben/Sicherheitsbestimmungen oder als weitere, einem Fachmann als sinnvoll erscheinende zentrale oder dezentrale Bedieneinheit, Eingabestation und/oder zentrales oder dezentrales Terminal ausgebildet ist. Es kann somit vorteilhaft eine Synchronisation von elektronischen Daten ermöglicht werden. Wird beispielsweise mittels eines Sensorelements der Sensorvorrichtung eine Anwesenheit eines Arbeitskleidungsteils der Arbeitskleidung erkannt, wird zumindest teilweise automatisch eine Verbindung zwischen der Kommunikationsvorrichtung und der externen Einheit aufgebaut. In der externen Einheit hinterlegte Einstellungen sind somit vorzugsweise direkt auf den Arbeitskleidungsteil der Arbeitskleidung und/oder von dem Arbeitskleidungsteil der Arbeitskleidung an die externe Einheit übertragbar. Hierbei kann es sich um individuelle Einstellungen oder um Vorgaben einer Firma handeln. Mittels der erfindungsgemäßen Ausgestaltung kann vorteilhaft eine individuelle Einstellung von Kenngrößen der Arbeitskleidung ermöglicht werden. Zudem kann vorteilhaft eine zentrale Steuerung von Funktionen der Arbeitskleidung und/oder des Aufbewahrungssystems ermöglicht werden. Ferner kann vorteilhaft während eines Ladevorgangs eine Aktualisierung einer Software erfolgen oder eine Erstellung einer Datensicherung von elektronischen Daten erfolgen. Hierbei ist es denkbar, dass zu einer Fehlervermeidung ein Öffnen des Aufbewahrungssystems unterbindbar ist, solang eine Datenübertragung noch nicht abgeschlossen ist und/oder ein Ladeprozess nicht beendet ist.

Des Weiteren wird vorgeschlagen, dass mittels der Kommunikationsvorrichtung elektronische Daten austauschbar sind, die eine Nutzungsdauer und/oder einen Abnutzungsgrad der Arbeitskleidung definieren, wobei die Nutzungsdauer und/oder der Abnutzungsgrad von zumindest einer Sensoreinheit der Arbeitskleidung und/oder von der Sensorvorrichtung erfassbar ist. Somit kann vorteilhaft nach einem Erreichen einer vorgegebenen maximalen Lebensdauer und/oder nach einem Erreichen eines maximalen Abnutzungsgrads vorteilhaft ein Ersetzen der Arbeitskleidung veranlasst werden. Hierdurch kann vorteilhaft sichergestellt werden, dass die Arbeitskleidung eine zuverlässige Schutzfunktion für einen Träger ermöglicht.

Ferner wird vorgeschlagen, dass mittels der Kommunikationsvorrichtung elektronische Daten austauschbar sind, die eine nutzerspezifische Kenngröße eines Trägers der Arbeitskleidung definieren, wobei die nutzerspezifische Kenngröße von zumindest einer Sensoreinheit der Arbeitskleidung erfassbar ist. Unter einer "nutzerspezifischen Kenngröße" soll hier insbesondere eine Kenngröße verstanden werden, die zumindest einen Vitalwert eines Trägers der Arbeitskleidung definiert, die abhängig ist von einem Verhalten und/oder von einer Tragedauer eines Trägers der Arbeitskleidung. Die nutzerspezifische Kenngröße kann hierbei als nutzerspezifische Arbeitskleidungsbelastung, als Trägerbelastung, wie beispielsweise eine Geräuschbelastung und/oder eine Schwingungsbelastung, als Puls eines Trägers der Arbeitskleidung, als Körpertemperatur eines Trägers der Arbeitskeildung, als Müdigkeitskenngröße eines Trägers der Arbeitskleidung, als Trägerausrichtungskenngröße, als Arbeitskleidungsbewegungskenngröße oder als eine andere, einem Fachmann als sinnvoll erscheinende nutzerspezifische Kenngröße ausgebildet sein. Mittels der erfindungsgemäßen Ausgestaltung kann vorteilhaft eine arbeitszeitabhängige Zusatzentlohnung eines Trägers realisiert werden. Es kann beispielsweise vorteilhaft eine während einer Schicht- oder Arbeitszeit von einem Träger der Arbeitskleidung aufgenommene Vibration (Vibrationsdosis) an eine zentrale Dienststelle über die Kommunikationsvorrichtung kommuniziert werden und damit können dem Träger entsprechende Zuschläge zukommen gelassen werden.

Erfindungsgemäss umfasst das Aufbewahrungssystem zumindest eine Steuer- und/oder Regelvorrichtung, die dazu vorgesehen ist, zumindest eine Kenngröße der Arbeitskleidung in Abhängigkeit von mittels der Kommunikationsvorrichtung ausgetauschten elektronischen Daten einzustellen. Unter einer "Steuer- und/oder Regelvorrichtung" soll insbesondere eine Vorrichtung mit zumindest einer Steuerelektronik verstanden werden. Unter einer "Steuerelektronik" soll insbesondere eine Einheit mit einer Prozessoreinheit und mit einer Speichereinheit sowie mit einem in der Speichereinheit gespeicherten Betriebsprogramm verstanden werden. Mittels der erfindungsgemäßen Ausgestaltung kann vorteilhaft eine Anpassung von Einstellungen der Arbeitskleidung an individuelle Bedürfnisse eines Trägers erfolgen, insbesondere während eines Ladeprozesses der Arbeitskleidung. Erfindungsgemäss wird eine Einstellung zumindest einer Kenngröße der Arbeitskleidung von einer zentralen Dienststelle erfolgen, und/oder ein Träger der Arbeitskleidung wird mittels eines Zusammenwirkens der Kommunikationsvorrichtung und einer externen Einheit eine Einstellung der zumindest einen Kenngröße vornehmen.

Des Weiteren wird vorgeschlagen, dass das Aufbewahrungssystem zumindest eine Trocknungs- und/oder Reinigungsvorrichtung umfasst, die dazu vorgesehen ist, in Abhängigkeit von zumindest einer mittels der Sensorvorrichtung erfassten Kenngröße und/oder in Abhängigkeit von mittels der Kommunikationsvorrichtung ausgetauschten elektronischen Daten einen Trocknungs- und/oder Reinigungsvorgang der Arbeitskleidung durchzuführen. Somit kann vorteilhaft ein zumindest teilautomatischer Trocknungs- und/oder Reinigungsvorgang erreicht werden. Somit kann vorteilhaft eine zuverlässige Einsetzbarkeit der Arbeitskleidung erfolgen.

Ferner wird vorgeschlagen, dass das Aufbewahrungssystem zumindest eine Ausgabevorrichtung zu einer Ausgabe von Informationen zumindest eines Zustands der Arbeitskleidung umfasst. Die Ausgabevorrichtung kann hierbei optisch, haptisch und/oder akustisch ausgebildet sein. Vorzugsweise ist die Ausgabevorrichtung zumindest als optische Ausgabevorrichtung ausgebildet. Mittels der erfindungsgemäßen Ausgestaltung kann vorteilhaft eine Statusmitteilung an einen Träger erfolgen, der somit vorteilhaft über einen Status der Arbeitskleidung, insbesondere in einem an der Aufbewahrungsvorrichtung angeordneten Zustand der Arbeitskleidung, informierbar ist.

In einer alternativen Ausgestaltung des Aufbewahrungssystems wird vorgeschlagen, dass zumindest die Aufbewahrungsvorrichtung mobil ausgeführt ist oder in einer mobilen Einheit integriert ist. Hierbei ist es denkbar, dass die Aufbewahrungsvorrichtung zumindest eine Mobilitätseinheit aufweist, mittels derer die Aufbewahrungsrichtung bewegbar ist. Die Mobilitätseinheit kann hierbei als Rolleneinheit, als Ketteneinheit, als Luftpolstereinheit, als Magneteinheit oder als eine andere, einem Fachmann als sinnvoll erscheinende Mobilitätseinheit ausgebildet sein. Die mobile Einheit ist vorzugsweise als Transportfahrzeug, wie beispielsweise als PKW, als NKW oder als LKW ausgebildet, in die die Aufbewahrungsvorrichtung integriert ist. Besonders bevorzugt sind alle Vorrichtungen des Aufbewahrungssystems mobil ausgeführt oder in einer mobilen Einheit integriert. Somit ist vorzugsweise das gesamte Aufbewahrungssystem mobil ausgeführt oder in einer mobilen Einheit integriert. Mittels der erfindungsgemäßen Ausgestaltung kann vorteilhaft eine hohe Flexibilität hinsichtlich eines Einsatzortes des Aufbewahrungssystems erreicht werden.

Zudem wird eine Ladevorrichtung, insbesondere für ein erfindungsgemäßes Aufbewahrungssystem vorgeschlagen, wobei die Ladevorrichtung zumindest dazu vorgesehen ist, elektrische Energie, insbesondere kontaktlos, an zumindest eine Energiespeichereinheit einer Arbeitskleidung zu übertragen. Vorzugsweise ist die Ladevorrichtung dazu vorgesehen, elektrische Energie, insbesondere kontaktlos, an verschiedene Arbeitskleidungsteile der Arbeitskleidung zu übertragen. Somit kann vorteilhaft eine Ladevorrichtung zur Verfügung gestellt werden, die ein großes Einsatzspektrum aufweist.

Das erfindungsgemäße Aufbewahrungssystem und/oder die erfindungsgemäße Ladevorrichtung sollen/soll hierbei nicht auf die oben beschriebene Anwendung und Ausführungsform beschränkt sein. Insbesondere können/kann das erfindungsgemäße Aufbewahrungssystem und/oder die erfindungsgemäße Ladevorrichtung zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

### Zeichnung

Weitere Vorteile ergeben sich aus der folgenden Zeichnungsbeschreibung. In der Zeichnung sind drei Ausführungsbeispiele der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein erfindungsgemäßes Aufbewahrungssystem mit einer Vielzahl miteinander verbundener Aufbewahrungsvorrichtungen in einer schematischen Darstellung,
- Fig. 2: eine Detailansicht einer Schutzhelmaufbewahrungseinheit einer der Aufbewahrungsvorrichtungen des erfindungsgemäßen Aufbewahrungssystems in einer schematischen Darstellung,
- Fig. 3: eine Detailansicht einer Handschuhaufbewahrungseinheit einer der Aufbewahrungsvorrichtungen des erfindungsgemäßen Aufbewahrungssystems in einer schematischen Darstellung,
- Fig. 4: eine Detailansicht einer Schutzbrillenaufbewahrungseinheit einer der Aufbewahrungsvorrichtungen des erfindungsgemäßen Aufbewahrungssystems in einer schematischen Darstellung,
- Fig. 5: eine Detailansicht einer Arbeitsjackenaufbewahrungseinheit einer der Aufbewahrungsvorrichtungen des erfindungsgemäßen Aufbewahrungssystems in einer schematischen Darstellung,
- Fig. 6: eine Detailansicht einer Arbeitsschuhaufbewahrungseinheit einer der Aufbewahrungsvorrichtungen des erfindungsgemäßen Aufbewahrungssystems in einer schematischen Darstellung,
- Fig. 7: eine weitere Detailansicht der Arbeitsschuhaufbewahrungseinheit einer der Aufbewahrungsvorrichtungen des erfindungsgemäßen Aufbewahrungssystems in einer schematischen Darstellung,
- Fig. 8: ein alternatives erfindungsgemäßes Aufbewahrungssystem in einer schematischen Darstellung und
- Fig. 9: ein weiteres, alternatives erfindungsgemäßes Aufbewahrungssystem in einer schematischen Darstellung.

### Beschreibung der Ausführungsbeispiele

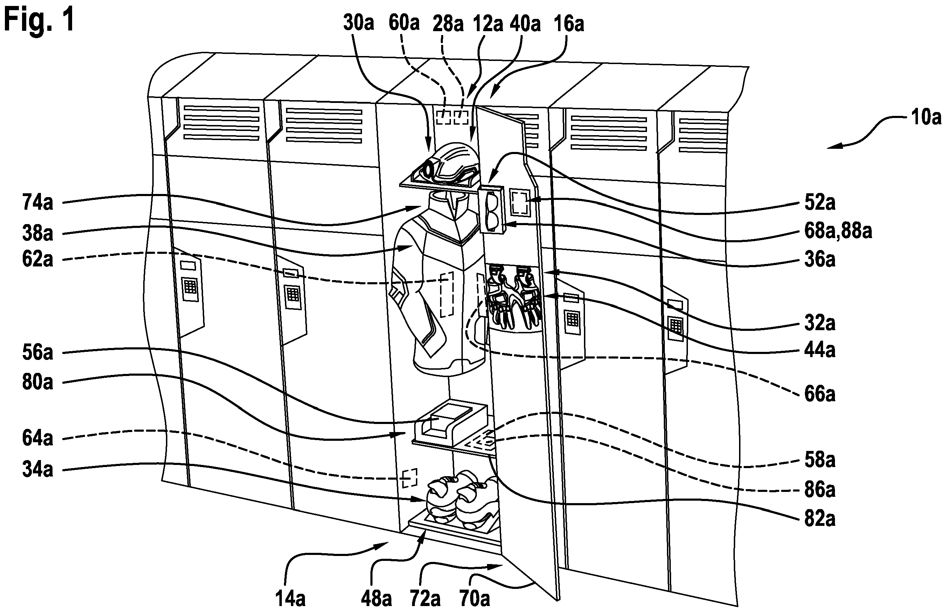

Figur 1 zeigt ein als Arbeitskleidungsaufbewahrungssystem ausgebildetes Aufbewahrungssystem 10a. Das Aufbewahrungssystem 10a umfasst zumindest eine Aufbewahrungsvorrichtung 12a zumindest zu einer Aufbewahrung von Arbeitskleidung 14a. Hierbei ist die Aufbewahrungsvorrichtung 12a als Spind ausgebildet. Insgesamt weist das Aufbewahrungssystem 10a eine Vielzahl an Aufbewahrungsvorrichtungen 12a auf, die eine zumindest im Wesentlichen analoge Ausgestaltung aufweisen. Somit ist das Aufbewahrungssystem 10a als Spindsystem ausgebildet. Bei dem in Figur 1 dargestellten Aufbewahrungssystem 10a sind die Aufbewahrungsvorrichtungen 12a direkt miteinander verbunden. Es ist jedoch auch denkbar, dass die Aufbewahrungsvorrichtungen 12a relativ zueinander beabstandet angeordnet sind, wobei eine Kommunikation der relativ zueinander beabstandeten Aufbewahrungsvorrichtungen 12a denkbar ist.

Ferner umfasst das Aufbewahrungssystem 10a zumindest eine kabelgebundene und/oder kabellose Ladevorrichtung 16a, die dazu vorgesehen ist, zumindest elektrische Energie an zumindest eine Energiespeichereinheit 18a, 20a, 22a, 24a, 26a der Arbeitskleidung 14a zu übertragen, und zumindest eine Sensorvorrichtung 28a zumindest zu einer Erfassung von zumindest einer Kenngröße der Arbeitskleidung 14a. Die Sensorvorrichtung 28a ist insbesondere zu einer Erfassung von zumindest einer Kenngröße der Arbeitskleidung 14a in einem an der Aufbewahrungsvorrichtung 12a angeordneten Zustand der Arbeitskleidung 14a vorgesehen. Die Ladevorrichtung 16a ist dazu vorgesehen, zumindest in einem an der Aufbewahrungsvorrichtung 12a angeordneten Zustand der Arbeitskleidung 14a zumindest elektrische Energie an zumindest eine Energiespeichereinheit 18a, 20a, 22a, 24a, 26a der Arbeitskleidung 14a zu übertragen. Die Ladevorrichtung 16a ist in die Aufbewahrungsvorrichtung 12a integriert. Die Aufbewahrungsvorrichtung 12a ist zu einer Aufbewahrung von verschiedenen Arbeitskleidungsteilen 30a, 32a, 34a, 36a, 38a der Arbeitskleidung 14a vorgesehen. Somit ist die Ladevorrichtung 16a dazu vorgesehen, verschiedene Arbeitskleidungsteile 30a, 32a, 34a, 36a, 38a der Arbeitskleidung 14a, die an der Aufbewahrungsvorrichtung 12a anordenbar sind, zu laden. Hierbei ist die Ladevorrichtung 16a insbesondere als Induktivladevorrichtung ausgebildet, wobei die Ladevorrichtung 16a eine Sekundärspuleneinheit aufweist und die Arbeitskleidung 14a, insbesondere die einzelnen Arbeitskleidungsteile 30a, 32a, 34a, 36a, 38a der Arbeitskleidung 14a, eine Primärspuleneinheit aufweist bzw. aufweisen. Die Ladevorrichtung 16a kann auch unabhängig von dem Aufbewahrungssystem 10a ausgebildet sein und eigenständig genutzt werden. Hierbei ist die Ladevorrichtung 16a zumindest dazu vorgesehen, elektrische Energie, insbesondere kontaktlos, an zumindest eine Energiespeichereinheit 18a, 20a, 22a, 24a, 26a einer Arbeitskleidung 14a zu übertragen.

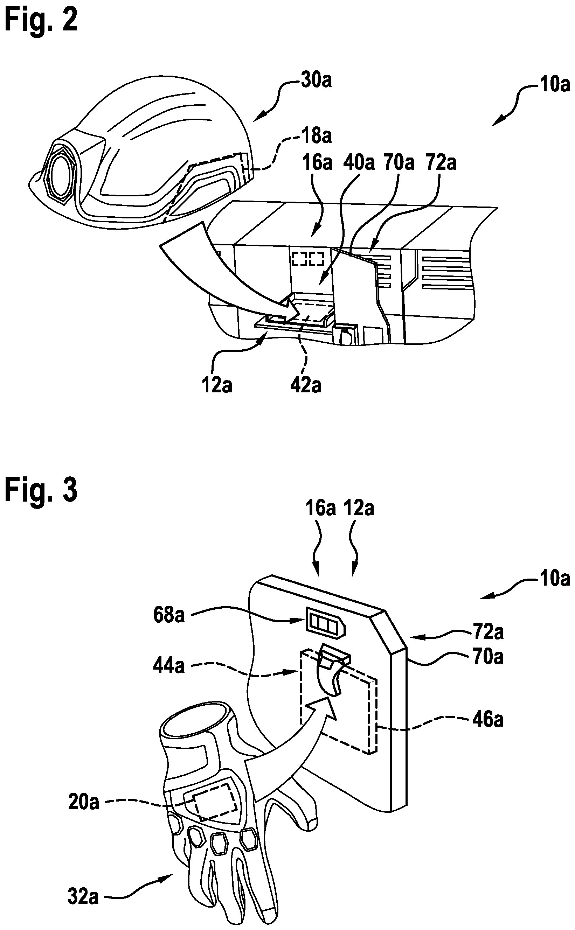

Die Aufbewahrungsvorrichtung 12a umfasst zumindest eine Schutzhelmaufbewahrungseinheit 40a, in der zumindest ein als Schutzhelm ausgebildetes Arbeitskleidungsteil 30a der Arbeitskleidung 14a anordenbar ist, wobei zumindest eine Schutzhelmladeeinheit 42a der Ladevorrichtung 16a zu einem Laden zumindest einer Energiespeichereinheit 18a des Schutzhelms an der Schutzhelmaufbewahrungseinheit 40a angeordnet ist (Figuren 1 und 2). Die Energiespeichereinheit 18a des Schutzhelms ist zu einer Energieversorgung von elektrischen und/oder elektronischen Komponenten des Schutzhelms vorgesehen, wie beispielsweise zu einer Energieversorgung einer Beleuchtungseinheit des Schutzhelm, einer Sensoreinheit des Schutzhelms, einer Datenübertragungseinheit des Schutzhelms, einer Ausgabeeinheit des Schutzhelms, einer aktiven Gehörschutzeinheit des Schutzhelms, einer Heizeinheit des Schutzhelms o. dgl. Die Schutzhelmaufbewahrungseinheit 40a ist als Schutzhelmaufbewahrungsschale vorgesehen, in der der Schutzhelm anordenbar ist. Hierbei ist die Schutzhelmaufbewahrungseinheit 40a in einem oberen Staufach der Aufbewahrungsvorrichtung 12a angeordnet. Es ist jedoch auch denkbar, dass die Schutzhelmaufbewahrungseinheit 40a eine andere, einem Fachmann als sinnvoll erscheinende Ausgestaltung und/oder Anordnung aufweist. Die Schutzhelmladeeinheit 42a ist als Induktivschutzhelmladeeinheit ausgebildet. Der Schutzhelm weist hierbei eine zu der Schutzhelmladeeinheit 42a korrespondierende Ladeeinheit auf. Somit ist die Energiespeichereinheit 18a des Schutzhelms in einem an der Schutzhelmaufbewahrungseinheit 40a angeordneten Zustand des Schutzhelms mittels eines Induktivladevorgangs aufladbar. Hierbei sind die Schutzhelmladeeinheit 42a und die Ladeeinheit des Schutzhelms infolge einer Anordnung des Schutzhelms an der Schutzhelmaufbewahrungseinheit 40a miteinander verbindbar, insbesondere kontaktlos miteinander verbindbar, wie beispielsweise induktiv miteinander verbindbar. Es ist jedoch auch denkbar, dass die Schutzhelmladeeinheit 42a als Kontaktladeeinheit ausgebildet ist und der Schutzhelm eine Gegenkontaktladeeinheit aufweist, die korrespondierend zueinander ausgebildet sind.

Des Weiteren umfasst die Aufbewahrungsvorrichtung 12a zumindest eine Handschuhaufbewahrungseinheit 44a, in der zumindest ein als Handschuhpaar ausgebildetes Arbeitskleidungsteil 32a der Arbeitskleidung 14a anordenbar ist, wobei zumindest eine Handschuhladeeinheit 46a der Ladevorrichtung 16a zu einem Laden zumindest einer Energiespeichereinheit 20a des Handschuhpaars an der Handschuhaufbewahrungseinheit 44a angeordnet ist (Figuren 1 und 3). Die Energiespeichereinheit 20a des Handschuhpaars ist zu einer Energieversorgung von elektrischen und/oder elektronischen Komponenten des Handschuhpaars vorgesehen, wie beispielsweise zu einer Energieversorgung einer Beleuchtungseinheit des Handschuhpaars, einer Sensoreinheit des Handschuhpaars, einer Datenübertragungseinheit des Handschuhpaars, einer Heizeinheit des Handschuhpaars o. dgl. Die Handschuhaufbewahrungseinheit 44a ist als Klemmund/oder Rasteinheit ausgebildet, mittels derer das Handschuhpaar an der Aufbewahrungsvorrichtung 12a anordenbar ist. Hierbei ist jeweils ein Handschuh des Handschuhpaars mittels eines Klemm- und/oder Rastelements der Handschuhaufbewahrungseinheit 44a an der Aufbewahrungsvorrichtung 12a anordenbar, insbesondere festklemmbar und/oder einrastbar. Die Handschuhaufbewahrungseinheit 44a ist an einem Verschlusselement 70a einer Verschlusseinheit 72a der Aufbewahrungsvorrichtung 12a, insbesondere an einer in einem verschlossenen Zustand der Aufbewahrungsvorrichtung 12a einem Aufbewahrungsraum der Aufbewahrungsvorrichtung 12a zugewandten Innenseite des Verschlusselements 70a, angeordnet. Das Verschlusselement 70a ist als schwenkbar gelagerte Spindtür ausgebildet, die dazu vorgesehen ist, den Aufbewahrungsraum der Aufbewahrungsvorrichtung 12a zu verschließen. Es ist jedoch auch denkbar, dass die Handschuhaufbewahrungseinheit 44a eine andere, einem Fachmann als sinnvoll erscheinende Ausgestaltung und/oder Anordnung aufweist, wie beispielsweise eine Ausgestaltung als Magnethalteeinheit o. dgl. Die Handschuhladeeinheit 46a ist als Induktivhandschuhladeeinheit ausgebildet. Das Handschuhpaar, insbesondere jeder Handschuh des Handschuhpaars, weist hierbei eine zu der Handschuhladeeinheit 46a korrespondierende Ladeeinheit auf. Somit ist die Energiespeichereinheit 20a des Handschuhpaars in einem an der Handschuhaufbewahrungseinheit 44a angeordneten Zustand des Handschuhpaars mittels eines Induktivladevorgangs aufladbar. Hierbei sind die Handschuhladeeinheit 46a und die Ladeeinheit des Handschuhpaars infolge einer Anordnung des Handschuhpaars an der Handschuhaufbewahrungseinheit 44a miteinander verbindbar, insbesondere kontaktlos miteinander verbindbar, wie beispielsweise induktiv miteinander verbindbar. Es ist jedoch auch denkbar, dass die Handschuhladeeinheit 46a als Kontaktladeeinheit ausgebildet ist und das Handschuhpaar eine Gegenkontaktladeeinheit aufweist, die korrespondierend zueinander ausgebildet sind.

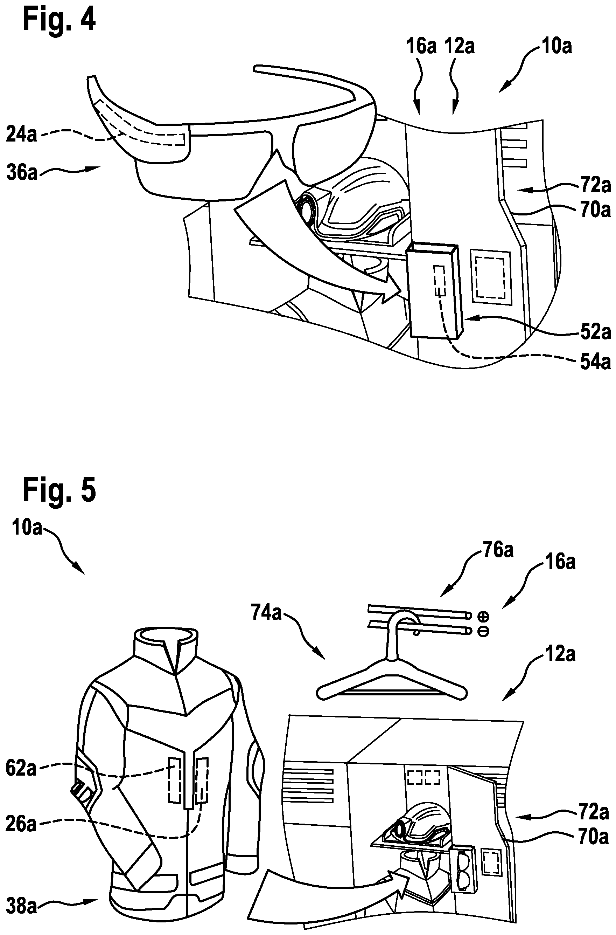

Ferner umfasst die Aufbewahrungsvorrichtung 12a zumindest eine Schutzbrillenaufbewahrungseinheit 52a, in der zumindest ein als Schutzbrille ausgebildetes Arbeitskleidungsteil 36a der Arbeitskleidung 14a anordenbar ist, wobei zumindest eine Schutzbrillenladeeinheit 54a der Ladevorrichtung 16a zu einer Energieversorgung zumindest einer Energiespeichereinheit 24a der Schutzbrille an der Schutzbrillenaufbewahrungseinheit 52a angeordnet ist (Figuren 1 und 4). Die Energiespeichereinheit 24a der Schutzbrille ist zu einer Energieversorgung von elektrischen und/oder elektronischen Komponenten der Schutzbrille vorgesehen, wie beispielsweise zu einer Energieversorgung einer Beleuchtungseinheit der Schutzbrille, einer Sensoreinheit der Schutzbrille, einer Datenübertragungseinheit der Schutzbrille, einer Ausgabeeinheit der Schutzbrille o. dgl. Die Schutzbrillenaufbewahrungseinheit 52a ist als Schutzbrillenaufnahmefach ausgebildet, mittels derer die Schutzbrille an der Aufbewahrungsvorrichtung 12a anordenbar ist. Hierbei ist die Schutzbrillenaufbewahrungseinheit 52a am Verschlusselement 70a, insbesondere an der in einem verschlossenen Zustand der Aufbewahrungsvorrichtung 12a dem Aufbewahrungsraum zugewandten Innenseite des Verschlusselements 70a, angeordnet. Es ist jedoch auch denkbar, dass die Schutzbrillenaufbewahrungseinheit 52a eine andere, einem Fachmann als sinnvoll erscheinende Ausgestaltung und/oder Anordnung aufweist. Die Schutzbrillenladeeinheit 54a ist als Induktivschutzbrillenladeeinheit ausgebildet. Die Schutzbrille weist hierbei eine zu der Schutzbrillenladeeinheit 54a korrespondierende Ladeeinheit auf. Somit ist die Energiespeichereinheit 24a der Schutzbrille in einem an der Schutzbrillenaufbewahrungseinheit 52a angeordneten Zustand der Schutzbrille mittels eines Induktivladevorgangs aufladbar. Hierbei sind die Schutzbrillenladeeinheit 54a und die Ladeeinheit der Schutzbrille infolge einer Anordnung der Schutzbrille an der Schutzbrillenaufbewahrungseinheit 52a miteinander verbindbar, insbesondere kontaktlos miteinander verbindbar, wie beispielsweise induktiv miteinander verbindbar. Es ist jedoch auch denkbar, dass die Schutzbrillenladeeinheit 54a als Kontaktladeeinheit ausgebildet ist und die Schutzbrille eine Gegenkontaktladeeinheit aufweist, die korrespondierend zueinander ausgebildet sind.

Zudem umfasst die Aufbewahrungsvorrichtung 12a zumindest eine Arbeitsjackenaufbewahrungseinheit 74a, an der zumindest ein als Arbeitsjacke ausgebildetes Arbeitskleidungsteil 38a der Arbeitskleidung 14a anordenbar ist, wobei zumindest eine Arbeitsjackenladeeinheit 76a der Ladevorrichtung 16a zu einem Laden zumindest einer Energiespeichereinheit 26a der Arbeitsjacke an der Arbeitsjackenaufbewahrungseinheit 74a angeordnet ist (Figuren 1 und 5). Die Energiespeichereinheit 26a der Arbeitsjacke ist zu einer Energieversorgung von elektrischen und/oder elektronischen Komponenten der Arbeitsjacke vorgesehen, wie beispielsweise zu einer Energieversorgung einer Beleuchtungseinheit der Arbeitsjacke, einer Sensoreinheit 62a der Arbeitsjacke, einer Datenübertragungseinheit der Arbeitsjacke, einer Ausgabeeinheit der Arbeitsjacke o. dgl. Die Arbeitsjackenaufbewahrungseinheit 74a ist als Kleiderbügel- und Kleiderstangeneinheit ausgebildet, mittels derer die Arbeitsjacke an der Aufbewahrungsvorrichtung 12a anordenbar ist. Es ist auch denkbar, dass die Arbeitsjackenaufbewahrungseinheit 74a alternativ oder zusätzlich zu einer Aufbewahrung eines als Arbeitshose oder als Arbeitsweste ausgebildeten Arbeitskleidungsteils (hier nicht näher dargestellt) der Arbeitskleidung 14a vorgesehen ist und/oder die Arbeitsjackenladeeinheit 76a zu einem Laden einer Energiespeichereinheit eines als Arbeitshose oder als Arbeitsweste ausgebildeten Arbeitskleidungsteils (hier nicht näher dargestellt) der Arbeitskleidung 14a vorgesehen ist. Die Arbeitsjackenaufbewahrungseinheit 74a ist im Aufbewahrungsraum der Aufbewahrungsvorrichtung 12a angeordnet. Es ist jedoch auch denkbar, dass die Arbeitsjackenaufbewahrungseinheit 74a eine andere, einem Fachmann als sinnvoll erscheinende Ausgestaltung und/oder Anordnung aufweist. Die Arbeitsjackenladeeinheit 76a ist als Induktivarbeitsjackenladeeinheit ausgebildet. Die Arbeitsjacke weist hierbei eine zu der Arbeitsjackenladeeinheit 76a korrespondierende Ladeeinheit auf. Somit ist die Energiespeichereinheit 26a der Arbeitsjacke in einem an der Arbeitsjackenaufbewahrungseinheit 74a angeordneten Zustand der Arbeitsjacke mittels eines Induktivladevorgangs aufladbar. Hierbei sind die Arbeitsjackenladeeinheit 76a und die Ladeeinheit der Arbeitsjacke infolge einer Anordnung der Arbeitsjacke an der Arbeitsjackenaufbewahrungseinheit 74a miteinander verbindbar, insbesondere kontaktlos miteinander verbindbar, wie beispielsweise induktiv miteinander verbindbar. Es ist jedoch auch denkbar, dass die Arbeitsjackenladeeinheit 76a als Kontaktladeeinheit ausgebildet ist und die Arbeitsjacke eine Gegenkontaktladeeinheit aufweist, die korrespondierend zueinander ausgebildet sind.

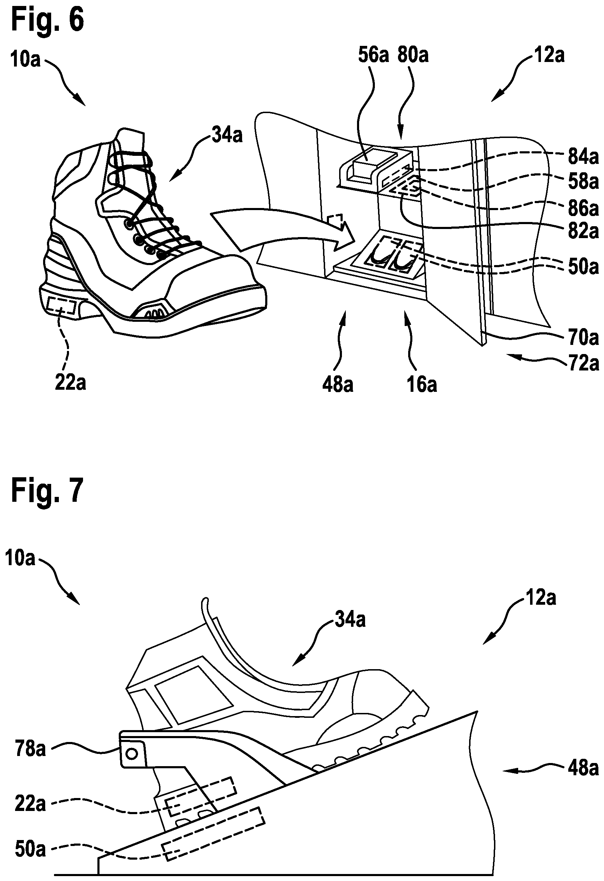

Des Weiteren umfasst die Aufbewahrungsvorrichtung 12a zumindest eine Arbeitsschuhaufbewahrungseinheit 48a, in der zumindest ein als Arbeitsschuhpaar ausgebildetes Arbeitskleidungsteil 34a der Arbeitskleidung 14a anordenbar ist, wobei zumindest eine Arbeitsschuhladeeinheit 50a der Ladevorrichtung 16a zu einem Laden zumindest einer Energiespeichereinheit 22a des Arbeitsschuhpaars an der Arbeitsschuhaufbewahrungseinheit 48a angeordnet ist (Figuren 1, 6 und 7). Die Energiespeichereinheit 22a des Arbeitsschuhpaars ist zu einer Energieversorgung von elektrischen und/oder elektronischen Komponenten des Arbeitsschuhpaars vorgesehen, wie beispielsweise zu einer Energieversorgung einer Beleuchtungseinheit des Arbeitsschuhpaars, einer Sensoreinheit des Arbeitsschuhpaars, einer Datenübertragungseinheit des Arbeitsschuhpaars, einer Ausgabeeinheit des Arbeitsschuhpaars o. dgl. Die Arbeitsschuhaufbewahrungseinheit 48a ist als Arbeitsschuhfach ausgebildet, in der das Arbeitsschuhpaar anordenbar ist. Die Arbeitsschuhaufbewahrungseinheit 48a weist zumindest ein bügelförmiges Arbeitsschuhhalteelement 78a auf, an dem ein Arbeitsschuh des Arbeitsschuhpaars in einem an der Arbeitsschuhaufbewahrungseinheit 48a angeordneten Zustand anliegt. Insgesamt weist die Arbeitsschuhaufbewahrungseinheit 48a zumindest zwei bügelförmige Arbeitsschuhhalteelemente 78a auf, wobei jeweils ein Arbeitsschuh des Arbeitsschuhpaars in einem an der Arbeitsschuhaufbewahrungseinheit 48a angeordneten Zustand an einem der bügelförmigen Arbeitsschuhhalteelemente 78a anliegt, insbesondere mit einem Fersenbereich des jeweiligen Arbeitsschuhs. Hierbei ist die Arbeitsschuhaufbewahrungseinheit 48a im Aufbewahrungsraum der Aufbewahrungsvorrichtung 12a angeordnet. Es ist jedoch auch denkbar, dass die Arbeitsschuhaufbewahrungseinheit 48a eine andere, einem Fachmann als sinnvoll erscheinende Ausgestaltung und/oder Anordnung aufweist.

Die Arbeitsschuhladeeinheit 50a ist als Induktivarbeitsschuhladeeinheit ausgebildet. Das Arbeitsschuhpaar, insbesondere jeder Arbeitsschuh des Arbeitsschuhpaars, weist hierbei eine zu der Arbeitsschuhladeeinheit 50a korrespondierende Ladeeinheit auf. Somit ist die Energiespeichereinheit 22a des Arbeitsschuhpaars in einem an der Arbeitsschuhaufbewahrungseinheit 48a angeordneten Zustand des Arbeitsschuhpaars mittels eines Induktivladevorgangs aufladbar. Die Arbeitsschuhladeeinheit 50a ist hierbei an den bügelförmigen Arbeitsschuhhalteelementen 78a oder in einer Bodenplatte der Arbeitsschuhladeeinheit 50a und/oder der Arbeitsschuhaufbewahrungseinheit 48a angeordnet. Die Arbeitsschuhladeeinheit 50a und die Ladeeinheit des Arbeitsschuhpaars sind infolge einer Anordnung des Arbeitsschuhpaars an der Arbeitsschuhaufbewahrungseinheit 48a miteinander verbindbar, insbesondere kontaktlos miteinander verbindbar, wie beispielsweise induktiv miteinander verbindbar. Es ist jedoch auch denkbar, dass die Arbeitsschuhladeeinheit 50a als Kontaktladeeinheit ausgebildet ist und das Arbeitsschuhpaar eine Gegenkontaktladeeinheit aufweist, die korrespondierend zueinander ausgebildet sind.

Ferner ist die Ladevorrichtung 16a dazu vorgesehen, zumindest eine Werkzeugmaschinenakkumulatorvorrichtung 56a und/oder zumindest eine Energiespeichereinheit einer externen Einheit 58a zu laden. Die Ladevorrichtung 16a ist dazu vorgesehen, zumindest eine Werkzeugmaschinenakkumulatorvorrichtung 56a und/oder zumindest eine Energiespeichereinheit einer externen Einheit 58a in einem an der Aufbewahrungsvorrichtung 12a angeordneten Zustand zu laden. Hierzu umfasst die Ladevorrichtung 16a zumindest eine Werkzeugmaschinenakkumulatorladeeinheit 84a und/oder zumindest eine Ladeeinheit 86a zu einem Laden der externen Einheit 58a. Zudem umfasst die Aufbewahrungsvorrichtung 12a zumindest eine Werkzeugmaschinenakkumulatoraufnahmeeinheit 80a und/oder zumindest eine Aufbewahrungseinheit 82a zu einem Aufbewahren der externen Einheit 58a. Die Werkzeugmaschinenakkumulatorladeeinheit 84a ist an der Werkzeugmaschinenakkumulatoraufnahmeeinheit 80a angeordnet. Die Ladeeinheit 86a zu einem Laden der externen Einheit 58a ist an der Aufbewahrungseinheit 82a zu einem Aufbewahren der externen Einheit 58a angeordnet. Hierbei sind/ist die Werkzeugmaschinenakkumulatorladeeinheit 84a und/oder die Ladeeinheit 86a zu einem Laden der externen Einheit 58a als Induktivladeeinheit ausgebildet. Es ist jedoch auch denkbar, dass die Werkzeugmaschinenakkumulatorladeeinheit 84a und/oder die Ladeeinheit 86a zu einem Laden der externen Einheit 58a als Kontaktladeeinheit ausgebildet sind/ist.

Zudem umfasst das Aufbewahrungssystem 10a zumindest eine Kommunikationsvorrichtung 60a zumindest zu einem Austausch von elektronischen Daten mit der Arbeitskleidung 14a. Die Kommunikationsvorrichtung 60a ist zu einem kabellosen Austausch von elektronischen Daten mit der Arbeitskleidung 14a vorgesehen. Hierbei sind mittels der Kommunikationsvorrichtung 60a elektronische Daten zwischen der Arbeitskleidung 14a und der externen Einheit 58a austauschbar. Zudem sind mittels der Kommunikationsvorrichtung 60a elektronische Daten austauschbar, die eine Nutzungsdauer und/oder einen Abnutzungsgrad der Arbeitskleidung 14a definieren, wobei die Nutzungsdauer und/oder der Abnutzungsgrad von zumindest der Sensoreinheit 62a der Arbeitskleidung 14a und/oder von der Sensorvorrichtung 28a erfassbar ist. Ferner sind mittels der Kommunikationsvorrichtung 60a elektronische Daten austauschbar, die eine nutzerspezifische Kenngröße eines Trägers der Arbeitskleidung 14a definieren, wobei die nutzerspezifische Kenngröße von zumindest der Sensoreinheit 62a der Arbeitskleidung 14a erfassbar ist.

Das Aufbewahrungssystem 10a umfasst des Weiteren zumindest eine Steuer- und/oder Regelvorrichtung 64a, die dazu vorgesehen ist, zumindest eine Kenngröße der Arbeitskleidung 14a in Abhängigkeit von einer individuellen Nutzereingabe und/oder in Abhängigkeit von mittels der Kommunikationsvorrichtung 60a ausgetauschten elektronischen Daten einzustellen. Somit kann eine individuelle Anpassung der Arbeitskleidung 14a an einen Träger und/oder an einen Einsatzbereich erfolgen, insbesondere zumindest teilautomatisch.

Ferner umfasst das Aufbewahrungssystem 10a zumindest eine Trocknungs- und/oder Reinigungsvorrichtung 66a, die dazu vorgesehen ist, in Abhängigkeit von zumindest einer mittels der Sensorvorrichtung 28a erfassten Kenngröße und/oder in Abhängigkeit von mittels der Kommunikationsvorrichtung 60a ausgetauschten elektronischen Daten einen Trocknungs- und/oder Reinigungsvorgang der Arbeitskleidung 14a durchzuführen. Hierbei ist es denkbar, dass die Sensoreinheit 62a der Arbeitskleidung 14a zumindest ein Feuchtesensorelement (hier nicht näher dargestellt) umfasst, mittels dessen eine als Feuchtigkeit der Arbeitskleidung 14a ausgebildete Kenngröße erfassbar ist, die mittels der Kommunikationsvorrichtung 60a an das Aufbewahrungssystem 10a übertragbar ist, wobei ein Trocknungs- und/oder Reinigungsvorgang in Abhängigkeit eines Werts der als Feuchtigkeit der Arbeitskleidung 14a ausgebildeten Kenngröße durchführbar ist. Zudem ist denkbar, dass der Trocknungs- und/oder Reinigungsvorgang in Abhängigkeit eines mittels einer Eingabevorrichtung 88a des Aufbewahrungssystems 10a eingegebenen Wunschs eines Trägers der Arbeitskleidung 14a durchführbar ist. Weitere, einem Fachmann als sinnvoll erscheinende Vorgänge zu einer Durchführung eines Trocknungs- und/oder Reinigungsvorgangs sind ebenfalls denkbar, wie beispielsweise infolge einer Kommunikation einer externen Einheit 58a mit dem Aufbewahrungssystem 10a mittels der Kommunikationsvorrichtung 60a o. dgl. Die Eingabevorrichtung 88a kann zudem zu einer Betätigung einer Verriegelungseinheit der Aufbewahrungsvorrichtung 12a vorgesehen sein.

Zudem umfasst das Aufbewahrungssystem 10a zumindest eine Ausgabevorrichtung 68a zu einer Ausgabe von Informationen zumindest eines Zustands der Arbeitskleidung 14a. Die Ausgabevorrichtung 68a kann hierbei als optische, haptische und/oder akustische Ausgabevorrichtung ausgebildet sein. Zudem ist es denkbar, dass die Ausgabevorrichtung 68a zumindest teilweise einteilig mit der Eingabevorrichtung 88a ausgebildet ist, insbesondere bei einer Ausgestaltung der Eingabevorrichtung 88a als berührungsempfindliche Displayvorrichtung. Die Ausgabevorrichtung 68a ist zumindest dazu vorgesehen, einen Status eines Ladevorgangs der Ladevorrichtung 16a und einen Ladestatus der Energiespeichereinheiten 18a, 20a, 22a, 24a, 26a der Arbeitskleidung 14a sowie der Werkzeugmaschinenakkumulatorvorrichtung 56a und einer Energiespeichereinheit einer externen Einheit 58a, insbesondere in einem an der Ladevorrichtung 16a und/oder an der Aufbewahrungsvorrichtung 12a angeordneten Zustand der Arbeitskleidung 14a sowie der Werkzeugmaschinenakkumulatorvorrichtung 56a und der externen Einheit 58a auszugeben. Hierbei weist die Ausgabevorrichtung 68a Ausgabeelemente auf, die an den entsprechenden Ladeeinheiten der Ladevorrichtung 16a angeordnet sind. Zudem ist die Ausgabevorrichtung 68a dazu vorgesehen, einen Status der Aufbewahrungsvorrichtung 12a auszugeben, wie beispielsweise einen Verschlussstatus, einen Trocknungs- und/oder Reinigungsstatus, einen Status darüber, welche Arbeitskleidungsteile 30a, 32a, 34a, 36a, 38a der Arbeitskleidung 14a an der Aufbewahrungsvorrichtung 12a angeordnet sind o. dgl. Hierzu ist zumindest ein optisches Ausgabeelement, insbesondere ein Leuchtelement, wie beispielsweise eine LED oder eine Lampe usw., der Ausgabevorrichtung 68a an einer Außenseite der Aufbewahrungsvorrichtung 12a angeordnet. Zudem ist eine berührungsempfindliche Displayvorrichtung der Ausgabevorrichtung 68a, die zumindest teilweise die Eingabevorrichtung 88a bildet, an einer Innenseite, insbesondere an einer Innenseite des Verschlusselements 70a, an der Aufbewahrungsvorrichtung 12a angeordnet. Weitere, einem Fachmann als sinnvoll erscheinende Ausgabeelemente der Ausgabevorrichtung 68a sind ebenfalls denkbar, wobei hier ein Fachmann, eine für Ihn sinnvolle Anordnung der weiteren Ausgabeelemente vornehmen wird.

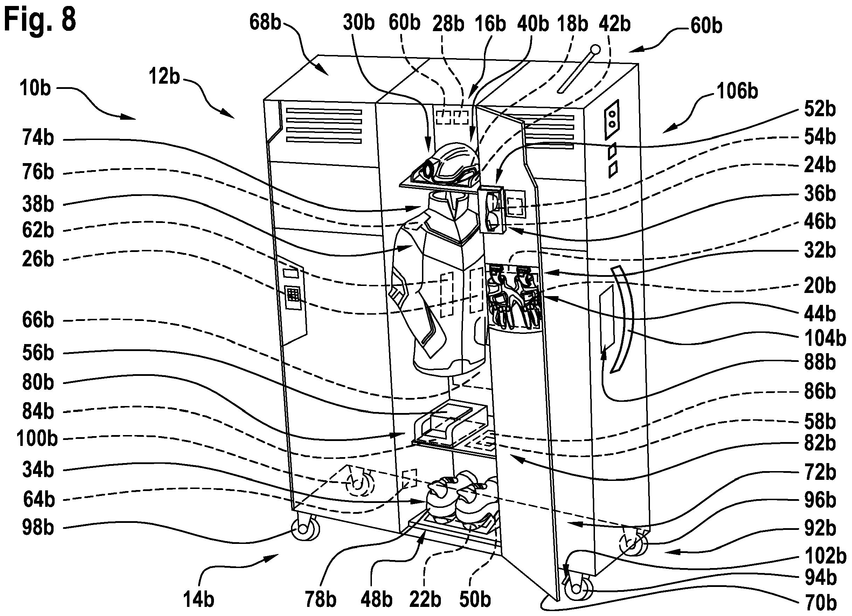

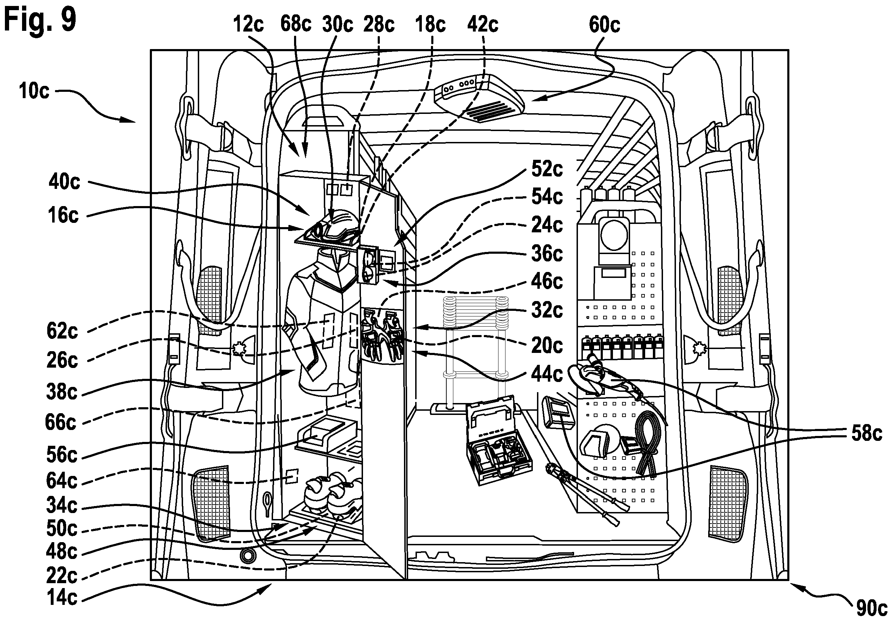

In Figuren 8 und 9 sind weitere Ausführungsbeispiele der Erfindung gezeigt. Die nachfolgenden Beschreibungen und die Zeichnung beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnung und/oder die Beschreibung der anderen Ausführungsbeispiele, insbesondere der Figuren 1 bis 7, verwiesen werden kann. Zur Unterscheidung der Ausführungsbeispiele ist der Buchstabe a den Bezugszeichen des Ausführungsbeispiels in den Figuren 1 bis 7 nachgestellt. In den Ausführungsbeispielen der Figuren 8 und 9 ist der Buchstabe a durch die Buchstaben b und c ersetzt.

Figur 8 zeigt ein alternatives Aufbewahrungssystem 10b, das zumindest eine Aufbewahrungsvorrichtung 12b zumindest zu einer Aufbewahrung von Arbeitskleidung 14b, zumindest eine kabelgebundene und/oder kabellose Ladevorrichtung 16b, die dazu vorgesehen ist, zumindest elektrische Energie an zumindest eine Energiespeichereinheit 18b, 20b, 22b, 24b, 26b der Arbeitskleidung 14b zu übertragen, und zumindest eine Sensorvorrichtung 28b zumindest zu einer Erfassung von zumindest einer Kenngröße der Arbeitskleidung 14b umfasst. Die Ladevorrichtung 16b ist hierbei dazu vorgesehen, verschiedene Arbeitskleidungsteile 30b, 32b, 34b, 36b, 38b der Arbeitskleidung 14b, die an der Aufbewahrungsvorrichtung 12b anordenbar sind, zu laden. Im Vergleich zu der in der Beschreibung der Figuren 1 bis 7 beschriebenen Aufbewahrungsvorrichtung 12a ist die in der Figur 8 dargestellte Aufbewahrungsvorrichtung 12b mobil ausgeführt. Hierbei umfasst die Aufbewahrungsvorrichtung 12b zumindest eine Mobilitätseinheit 92b. Die Mobilitätseinheit 92b umfasst hierbei zumindest eine Rolle 94b, mittels derer die Aufbewahrungsvorrichtung 12b bewegbar ist. Insgesamt umfasst die Mobilitätseinheit 92b vier Rollen 94b, 96b, 98b, 100b. Hierbei ist jeweils eine Rolle 94b, 96b, 98b, 100b der Rollen 94b, 96b, 98b, 100b an einem Randbereich, insbesondere an einer Ecke, der Aufbewahrungsvorrichtung 12b angeordnet. Die Mobilitätseinheit 92b umfasst ferner zumindest eine Bremseinheit 102b zu einer Blockierung einer Bewegungsmöglichkeit zumindest einer der Rollen 94b, 96b, 98b, 100b. Die Bremseinheit 102b ist hierbei als Reibbremseinheit ausgebildet, die zumindest ein als Bremsklotz ausgebildetes Bremselement (hier nicht näher dargestellt) aufweist. Ferner umfasst die Mobilitätseinheit 92b zumindest ein Haltegriff 104b. Alternativ oder zusätzlich ist es denkbar, dass die Mobilitätseinheit 92b zumindest eine Antriebseinheit umfasst, mittels derer die Aufbewahrungsvorrichtung 12b zumindest teilweise automatisch bewegbar ist. Somit ist es denkbar, dass die Aufbewahrungsvorrichtung 12b, ähnlich wie ein RC-Modell, fernsteuerbar ausgebildet ist. Weitere, einem Fachmann als sinnvoll erscheinende Ausgestaltungen der Mobilitätseinheit 92b sind ebenfalls denkbar. Alle weiteren Vorrichtungen und Einheiten des Aufbewahrungssystems 10b sind hierbei an der Aufbewahrungsvorrichtung 12b angeordnet oder in diese integriert, wie bereits in der Beschreibung der Figuren 1 bis 7 beschrieben. Zu einer Versorgung der Ladevorrichtung 16b mit elektrischer Energie weist das Aufbewahrungssystem 10b zumindest eine Energieanschlussvorrichtung 106b auf. Die Energieanschlussvorrichtung 106b weist hierbei eine, einem Fachmann bereits bekannte Ausgestaltung auf. Hinsichtlich weiterer Funktionen und Merkmale des in der Figur 8 dargestellten Aufbewahrungssystems 10b darf auf das in den Figuren 1 bis 7 dargestellte Aufbewahrungssystem 10a verwiesen werden.

Figur 9 zeigt ein weiteres alternatives Aufbewahrungssystem 10c, das zumindest eine Aufbewahrungsvorrichtung 12c zumindest zu einer Aufbewahrung von Arbeitskleidung 14c, zumindest eine kabelgebundene und/oder kabellose Ladevorrichtung 16c, die dazu vorgesehen ist, zumindest elektrische Energie an zumindest eine Energiespeichereinheit 18c, 20c, 22c, 24c, 26c der Arbeitskleidung 14c zu übertragen, und zumindest eine Sensorvorrichtung 28c zumindest zu einer Erfassung von zumindest einer Kenngröße der Arbeitskleidung 14c umfasst. Die Ladevorrichtung 16c ist hierbei dazu vorgesehen, verschiedene Arbeitskleidungsteile 30c, 32c, 34c, 36c, 38c der Arbeitskleidung 14c, die an der Aufbewahrungsvorrichtung 12c anordenbar sind, zu laden. Im Vergleich zu der in der Beschreibung der Figuren 1 bis 7 beschriebenen Aufbewahrungsvorrichtung 12a ist die in der Figur 9 dargestellte Aufbewahrungsvorrichtung 12c in eine mobile Einheit 90c integriert. Die mobile Einheit 90c ist hierbei als NKW (Nutzkraftwagen) ausgebildet. Es ist jedoch auch denkbar, dass die mobile Einheit 90c eine andere, einem Fachmann als sinnvoll erscheinende Ausgestaltung aufweist, wie beispielsweise als PKW, als LKW o. dgl. Alle weiteren Vorrichtungen und Einheiten des Aufbewahrungssystems 10c können hierbei an der Aufbewahrungsvorrichtung 12c angeordnet, in diese integriert sein oder einzeln in die mobile Einheit 90c integriert sein. Hinsichtlich weiterer Funktionen und Merkmale des in der Figur 8 dargestellten Aufbewahrungssystems 10c darf auf das in den Figuren 1 bis 7 dargestellte Aufbewahrungssystem 10a verwiesen werden.

## Patentansprüche

1. Aufbewahrungssystem, insbesondere Arbeitskleidungsaufbewahrungssystem, mit zumindest einer Aufbewahrungsvorrichtung (12a; 12b; 12c) zumindest zu einer Aufbewahrung von Arbeitskleidung (14a; 14b; 14c), mit zumindest einer kabelgebundenen und/oder kabellosen Ladevorrichtung (16a; 16b; 16c), die dazu vorgesehen ist, zumindest elektrische Energie an zumindest eine Energiespeichereinheit (18a, 20a, 22a, 24a, 26a; 18b, 20b, 22b, 24b, 26b; 18c, 20c, 22c, 24c, 26c) der Arbeitskleidung (14a; 14b; 14c) zu übertragen, mit zumindest einer Sensorvorrichtung (28a; 28b; 28c) zumindest zu einer Erfassung von zumindest einer Kenngröße der Arbeitskleidung (14a; 14b; 14c), wobei die Ladevorrichtung (16a; 16b; 16c) dazu vorgesehen ist, verschiedene Arbeitskleidungsteile (30a, 32a, 34a, 36a, 38a; 30b, 32b, 34b, 36b, 38b; 30c, 32c, 34c, 36c, 38c; 30c) der Arbeitskleidung (14a; 14b; 14c), die an der Aufbewahrungsvorrichtung (12a; 12b; 12c) anordenbar sind, zu laden, und mit zumindest einer Kommunikationsvorrichtung (60a; 60b; 60c) zumindest zu einem Austausch von elektronischen Daten mit der Arbeitskleidung (14a; 14b; 14c), **gekennzeichnet durch** zumindest eine Steuer- und/oder Regelvorrichtung (64a; 64b; 64c), die dazu vorgesehen ist, zumindest eine Kenngröße der Arbeitskleidung (14a; 14b; 14c) mittels über die Kommunikationsvorrichtung (60a; 60b; 60c) ausgetauschter elektronischer Daten einzustellen, wobei die zumindest eine Kenngröße der Arbeitskleidung (14a; 14b; 14c) von einer mit der Kommunikationsvorrichtung verbundenen zentralen Dienststelle einstellbar ist und alternativ die zumindest eine Kenngröße durch einen Träger der Arbeitskleidung (14a; 14b; 14c) mittels einer mit der Kommunikationsvorrichtung (60a; 60b; 60c) zusammenwirkenden externen Einheit (58a; 58b; 58c) einstellbar ist.

2. Aufbewahrungssystem nach Anspruch 1, **dadurch gekennzeichnet, dass** die Aufbewahrungsvorrichtung (12a; 12b; 12c) zumindest eine Schutzhelmaufbewahrungseinheit (40a; 40b; 40c) umfasst, in der zumindest ein als Schutzhelm ausgebildetes Arbeitskleidungsteil (30a; 30b; 30c) der Arbeitskleidung (14a; 14b; 14c) anordenbar ist, wobei zumindest eine Schutzhelmladeeinheit (42a; 42b; 42c) der Ladevorrichtung (16a; 16b; 16c) zu einem Laden zumindest einer Energiespeichereinheit (18a; 18b; 18c) des Schutzhelms an der Schutzhelmaufbewahrungseinheit (40a; 40b; 40c) angeordnet ist.

3. Aufbewahrungssystem nach einem der vorhergehenden Ansprüche, **dadurch gekennzeichnet, dass** die Aufbewahrungsvorrichtung (12a; 12b; 12c) zumindest eine Handschuhaufbewahrungseinheit (44a; 44b; 44c)
umfasst, in der zumindest ein als Handschuhpaar ausgebildetes Arbeitskleidungsteil (32a; 32b; 32c) der Arbeitskleidung (14a; 14b; 14c) anordenbar ist, wobei zumindest eine Handschuhladeeinheit (46a; 46b; 46c) der Ladevorrichtung (16a; 16b; 16c) zu einem Laden zumindest einer Energiespeichereinheit (20a; 20b; 20c) des Handschuhpaars an der Handschuhaufbewahrungseinheit (44a; 44b; 44c) angeordnet ist.

4. Aufbewahrungssystem nach einem der vorhergehenden Ansprüche, **dadurch gekennzeichnet, dass** die Aufbewahrungsvorrichtung (12a; 12b; 12c) zumindest eine Arbeitsschuhaufbewahrungseinheit (48a; 48b; 48c) umfasst, in der zumindest ein als Arbeitsschuhpaar ausgebildetes Arbeitskleidungsteil (34a; 34b; 34c) der Arbeitskleidung (14a; 14b; 14c) anordenbar ist, wobei zumindest eine Arbeitsschuhladeeinheit (50a; 50b; 50c) der Ladevorrichtung (16a; 16b; 16c) zu einem Laden zumindest einer Energiespeichereinheit (22a; 22 ; 22c) des Arbeitsschuhpaars an der Arbeitsschuhaufbewahrungseinheit (48a; 48b; 48c) angeordnet ist.

5. Aufbewahrungssystem nach einem der vorhergehenden Ansprüche, **dadurch gekennzeichnet, dass** die Aufbewahrungsvorrichtung (12a; 12b; 12c) zumindest eine Schutzbrillenaufbewahrungseinheit (52a; 52b; 52c) umfasst, in der zumindest eine als Schutzbrille ausgebildetes Arbeitskleidungsteil (36a; 36b; 36c) der Arbeitskleidung (14a; 14b; 14c) anordenbar ist, wobei zumindest eine Schutzbrillenladeeinheit (54a ; 54b; 54c) der Ladevorrichtung (16a; 16b; 16c) zu einer Energieversorgung zumindest einer Energiespeichereinheit (24a; 24b; 24c) der Schutzbrille an der Schutzbrillenaufbewahrungseinheit (52a; 52b; 52c) angeordnet ist.

6. Aufbewahrungssystem nach einem der vorhergehenden Ansprüche, **dadurch gekennzeichnet, dass** die Ladevorrichtung (16a; 16b; 16c) dazu vorgesehen ist, zumindest eine Werkzeugmaschinenakkumulatorvorrichtung (56a; 56b; 56c) und/oder zumindest eine Energiespeichereinheit einer externen Einheit (58a; 58b; 58c) zu laden.

7. Aufbewahrungssystem nach einem der vorhergehenden Ansprüche, **dadurch gekennzeichnet, dass** mittels der Kommunikationsvorrichtung (60a; 60b; 60c) elektronische Daten zwischen der Arbeitskleidung (14a; 14b; 14c) und einer externen Einheit (58a; 58b; 58c) austauschbar sind.

8. Aufbewahrungssystem nach einem der vorhergehenden Ansprüche, **dadurch gekennzeichnet, dass** mittels der Kommunikationsvorrichtung (60a; 60b; 60c) elektronische Daten austauschbar sind, die eine Nutzungsdauer und/oder einen Abnutzungsgrad der Arbeitskleidung (14a; 14b; 14c) definieren, wobei die Nutzungsdauer und/oder der Abnutzungsgrad von zumindest einer Sensoreinheit (62a; 62b; 62c) der Arbeitskleidung (14a; 14b; 14c) und/oder von der Sensorvorrichtung (28a; 28b; 28c) erfassbar ist.

9. Aufbewahrungssystem nach einem der vorhergehenden_Ansprüche, **dadurch gekennzeichnet, dass** mittels der Kommunikationsvorrichtung (60a; 60b; 60c) elektronische Daten austauschbar sind, die eine nutzerspezifische Kenngröße eines Trägers der Arbeitskleidung (14a; 14b; 14c) definieren, wobei die nutzerspezifische Kenngröße von zumindest einer Sensoreinheit (62a; 62b; 62c) der Arbeitskleidung (14a; 14b; 14c) erfassbar ist.

10. Aufbewahrungssystem nach einem der vorhergehenden Ansprüche, **gekennzeichnet durch** zumindest eine Trocknungs- und/oder Reinigungsvorrichtung (66a; 66b; 66c), die dazu vorgesehen ist, in Abhängigkeit von zumindest einer mittels der Sensorvorrichtung (28a; 28a; 28c) erfassten Kenngröße und/oder in Abhängigkeit von mittels der Kommunikationsvorrichtung (60a; 60b; 60c) ausgetauschten elektronischen Daten einen Trocknungs- und/oder Reinigungsvorgang der Arbeitskleidung (14a; 14b; 14c) durchzuführen.

11. Aufbewahrungssystem nach einem der vorhergehenden Ansprüche, **gekennzeichnet durch** zumindest eine Ausgabevorrichtung (68a; 68b; 68c) zu einer Ausgabe von Informationen zumindest eines Zustands der Arbeitskleidung (14a; 14b; 14c).

12. Aufbewahrungssystem nach einem der vorhergehenden Ansprüche, **dadurch gekennzeichnet, dass** zumindest die Aufbewahrungsvorrichtung (12b; 12c) mobil ausgeführt ist oder in einer mobilen Einheit (90c) integriert ist.

## Claims

1. Storage system, in particular work-clothing storage system, comprising at least one storage device (12a; 12b; 12c) at least for storing work clothing (14a; 14b; 14c), comprising at least one wired and/or wireless charging device (16a; 16b; 16c) which is provided for transmitting at least electrical energy to at least one energy storage unit (18a, 20a, 22a, 24a, 26a; 18b, 20b, 22b, 24b, 26b; 18c, 20c, 22c, 24c, 26c) of the work clothing (14a; 14b; 14c), comprising at least one sensor device (28a; 28b; 28c) at least for sensing at least one characteristic variable of the work clothing (14a; 14b; 14c), wherein the charging device (16a; 16b; 16c) is provided for charging different work-clothing items (30a, 32a, 34a, 36a, 38a; 30b, 32b, 34b, 36b, 38b; 30c, 32c, 34c, 36c, 38c; 30c) of the work clothing (14a; 14b; 14c), which can be disposed on the storage device (12a; 12b; 12c), and comprising at least one communication device (60a; 60b; 60c) at least for exchanging electronic data with the work clothing (14a; 14b; 14c), **characterized by** at least one control and/or regulating device (64a; 64b; 64c) which is provided for setting at least one characteristic variable of the work clothing (14a; 14b; 14c) by means of electronic data exchanged via the communication device (60a; 60b; 60c), wherein the at least one characteristic variable of the work clothing (14a; 14b; 14c) can be set by a centralized service point connected to the communication device, and alternatively the at least one characteristic variable can be set by a wearer of the work clothing (14a; 14b; 14c) by means of an external unit (58a; 58b; 58c) that interacts with the communication device (60a; 60b; 60c) .

2. Storage system according to Claim 1, **characterized in that** the storage device (12a; 12b; 12c) comprises at least one safety helmet storage unit (40a; 40b; 40c), in which at least one work-clothing item (30a; 30b; 30c) of the work clothing (14a; 14b; 14c), which is designed as a safety helmet, can be disposed, wherein at least one safety helmet charging unit (42a; 42b; 42c) of the charging device (16a; 16b; 16c) is disposed on the safety helmet storage unit (40a; 40b; 40c) for charging at least one energy storage unit (18a; 18b; 18c) of the safety helmet.

3. Storage system according to one of the preceding claims, **characterized in that** the storage device (12a; 12b; 12c) comprises at least one glove storage unit (44a; 44b; 44c), in which at least one work-clothing item (32a; 32b; 32c) of the work clothing (14a; 14b; 14c), which is designed as a pair of gloves, can be disposed, wherein at least one glove charging unit (46a; 46b; 46c) of the charging device (16a; 16b; 16c) is disposed on the glove storage unit (44a; 44b; 44c) for charging at least one energy storage unit (20a; 20b; 20c) of the pair of gloves.

4. Storage system according to one of the preceding claims, **characterized in that** the storage device (12a; 12b; 12c) comprises at least one work shoe storage unit (48a; 48b; 48c), in which at least one work-clothing item (34a; 34b; 34c) of the work clothing (14a; 14b; 14c), which is designed as a pair of work shoes, can be disposed, wherein at least one work shoe charging unit (50a; 50b; 50c) of the charging device (16a; 16b; 16c) is disposed on the work shoe storage unit (48a; 48b; 48c) for charging at least one energy storage unit (22a; 22; 22c) of the pair of work shoes.

5. Storage system according to one of the preceding claims, **characterized in that** the storage device (12a; 12b; 12c) comprises at least one safety glasses storage unit (52a; 52b; 52c), in which at least one work-clothing item (36a; 36b; 36c) of the work clothing (14a; 14b; 14c), which is designed as safety glasses, can be disposed, wherein at least one safety glasses charging unit (54a; 54b; 54c) of the charging device (16a; 16b; 16c) is disposed on the safety glasses storage unit (52a; 52b; 52c) for supplying at least one energy storage unit (24a; 24b; 24c) of the safety glasses with energy.

6. Storage system according to one of the preceding claims, **characterized in that** the charging device (16a; 16b; 16c) is provided for charging at least one machine tool accumulator device (56a; 56b; 56c) and/or at least one energy storage unit of an external unit (58a; 58b; 58c) .

7. Storage system according to one of the preceding claims, **characterized in that** electronic data can be exchanged between the work clothing (14a; 14b; 14c) and an external unit (58a; 58b; 58c) by means of the communication device (60a; 60b; 60c).

8. Storage system according to one of the preceding claims, **characterized in that** electronic data which define a usage duration and/or a degradation of the work clothing (14a; 14b; 14c) can be exchanged by means of the communication device (60a; 60b; 60c), wherein the usage duration and/or the degradation can be sensed by at least one sensor unit (62a; 62b; 62c) of the work clothing (14a; 14b; 14c) and/or by the sensor device (28a; 28b; 28c).

9. Storage system according tos one of the preceding claims, **characterized in that** electronic data which define a user-specific characteristic variable of a wearer of the work clothing (14a; 14b; 14c) can be exchanged by means of the communication device (60a; 60b; 60c), wherein the user-specific characteristic variable can be sensed by at least one sensor unit (62a; 62b; 62c) of the work clothing (14a; 14b; 14c).

10. Storage system according to one of the preceding claims, **characterized by** at least one drying and/or cleaning device (66a; 66b; 66c) which is provided for carrying out a drying and/or cleaning process of the work clothing (14a; 14b; 14c) depending on at least one characteristic variable sensed by means of the sensor device (28a; 28a; 28c) and/or depending on electronic data exchanged by means of the communication device (60a; 60b; 60c).

11. Storage system according to one of the preceding claims, **characterized by** at least one output device (68a; 68b; 68c) for outputting information at least regarding a state of the work clothing (14a; 14b; 14c).

12. Storage system according to one of the preceding claims, **characterized in that** at least the storage device (12b; 12c) is designed to be mobile or is integrated in a mobile unit (90c).

## Revendications

1. Système d'entreposage, en particulier système d'entreposage de vêtements de travail, avec au moins un dispositif d'entreposage (12a ; 12b ; 12c) au moins pour un entreposage de vêtements de travail (14a ; 14b ; 14c), avec au moins un dispositif de charge connecté par câble et/ou sans câble (16a ; 16b ; 16c), qui est prévu au moins pour transférer de l'énergie électrique à au moins une unité d'accumulation d'énergie (18a, 20a, 22a, 24a, 26a ; 18b, 20b, 22b, 24b, 26b ; 18c, 20c, 22c, 24c, 26c) des vêtements de travail (14a ; 14b ; 14c), avec au moins un dispositif de capteurs (28a ; 28b ; 28c) au moins en vue d'une détection d'au moins une grandeur caractéristique des vêtements de travail (14a ; 14b ; 14c), dans lequel le dispositif de charge (16a ; 16b ; 16c) est prévu pour charger différentes pièces des vêtements de travail (30a, 32a, 34a, 36a, 38a ; 30b, 32b, 34b, 36b, 38b ; 30c, 32c, 34c, 36c, 38c ; 30c) des vêtements de travail (14a ; 14b ; 14c), qui peuvent être disposées sur le dispositif d'entreposage (12a ; 12b ; 12c), et avec au moins un dispositif de communication (60a ; 60b ; 60c) au moins en vue d'un échange de données électroniques avec les vêtements de travail (14a ; 14b ; 14c), **caractérisé par** au moins un dispositif de commande et/ou de régulation (64a ; 64b ; 64c), qui est prévu au moins pour régler une grandeur caractéristique des vêtements de travail (14a ; 14b ; 14c) au moyen de données électroniques échangées par le dispositif de communication (60a ; 60b ; 60c), dans lequel ladite au moins une grandeur caractéristique des vêtements de travail (14a ; 14b ; 14c) peut être réglée à partir d'un poste de commande central relié au dispositif de communication et en variante ladite au moins une grandeur caractéristique peut être réglée par un support des vêtements de travail (14a ; 14b ; 14c) au moyen d'une unité externe (58a ; 58b ; 58c) coopérant avec le dispositif de communication (60a ; 60b ; 60c).

2. Système d'entreposage selon la revendication 1, **caractérisé en ce que** le dispositif d'entreposage (12a ; 12b ; 12c) comprend au moins une unité d'entreposage de casque de protection (40a ; 40b ; 40c), dans laquelle au moins une pièce de vêtement de travail sous la forme d'un casque de protection (30a ; 30b ; 30c) des vêtements de travail (14a ; 14b ; 14c) peut être disposée, dans lequel au moins une unité de charge de casque de protection (42a ; 42b ; 42c) du dispositif de charge (16a ; 16b ; 16c) est disposée en vue d'une charge d'au moins une unité d'accumulation d'énergie (18a ; 18b ; 18c) du casque de protection à l'unité d'entreposage de casque de protection (40a ; 40b ; 40c).

3. Système d'entreposage selon l'une quelconque des revendications précédentes, **caractérisé en ce que** le dispositif d'entreposage (12a ; 12b ; 12c) comprend au moins une unité d'entreposage de gants (44a ; 44b ; 44c), dans laquelle au moins une pièce de vêtement de travail sous la forme d'une paire de gants (32a ; 32b ; 32c) des vêtements de travail (14a ; 14b ; 14c) peut être disposée, dans lequel au moins une unité de charge de gants (46a ; 46b ; 46c) du dispositif de charge (16a ; 16b ; 16c) est disposée en vue d'une charge d'au moins une unité d'accumulation d'énergie (20a ; 20b ; 20c) de la paire de gants à l'unité d'entreposage de gants (44a ; 44b ; 44c).

4. Système d'entreposage selon l'une quelconque des revendications précédentes, **caractérisé en ce que** le dispositif d'entreposage (12a ; 12b ; 12c) comprend une unité d'entreposage de chaussures de travail (48a ; 48b ; 48c), dans laquelle au moins une pièce de vêtement de travail sous la forme d'une paire de chaussures de travail (34a ; 34b ; 34c) des vêtements de travail (14a ; 14b ; 14c) peut être disposée, dans lequel au moins une unité de charge de chaussures de travail (50a ; 50b ; 50c) du dispositif de charge (16a ; 16b ; 16c) est disposée en vue d'une charge d'au moins une unité d'accumulation d'énergie (22a ; 22b ; 22c) de la paire de chaussures de travail à l'unité d'entreposage de chaussures de travail (48a ; 48b ; 48c).

5. Système d'entreposage selon l'une quelconque des revendications précédentes, **caractérisé en ce que** le dispositif d'entreposage (12a ; 12b ; 12c) comprend au moins une unité d'entreposage de lunettes de protection (52a ; 52b ; 52c), dans laquelle au moins une pièce de vêtement de travail sous la forme de lunettes de protection (36a ; 36b ; 36c) des vêtements de travail (14a ; 14b ; 14c) peut être disposée, dans lequel au moins une unité de charge de lunettes de protection (54a ; 54b ; 54c) du dispositif de charge (16a ; 16b ; 16c) est disposée en vue d'une alimentation en énergie d'au moins une unité d'accumulation d'énergie (24a ; 24b ; 24c) des lunettes de protection à l'unité d'entreposage de lunettes de protection (52a ; 52b ; 52c) .

6. Système d'entreposage selon l'une quelconque des revendications précédentes, **caractérisé en ce que** le dispositif de charge (16a ; 16b ; 16c) est prévu pour charger au moins un dispositif d'accumulateur de machineoutil (56a ; 56b ; 56c) et/ou au moins une unité d'accumulation d'énergie d'une unité externe (58a ; 58b ; 58c) .

7. Système d'entreposage selon l'une quelconque des revendications précédentes, **caractérisé en ce que** des données électroniques peuvent être échangées entre les vêtements de travail (14a ; 14b ; 14c) et une unité externe (58a ; 58b ; 58c) au moyen du dispositif de communication (60a ; 60b ; 60c).

8. Système d'entreposage selon l'une quelconque des revendications précédentes, **caractérisé en ce que** des données électroniques peuvent être échangées au moyen du dispositif de communication (60a ; 60b ; 60c), qui définissent une durée d'utilisation et/ou un degré d'usure des vêtements de travail (14a ; 14b ; 14c), dans lequel la durée d'utilisation et/ou le degré d'usure est détectable par au moins une unité de capteurs (62a ; 62b ; 62c) des vêtements de travail (14a ; 14b ; 14c) et/ou par le dispositif de capteurs (28a ; 28b ; 28c).

9. Système d'entreposage selon l'une quelconque des revendications précédentes, **caractérisé en ce que** des données électroniques peuvent être échangées au moyen du dispositif de communication (60a ; 60b ; 60c), qui définissent une grandeur caractéristique spécifique à l'utilisateur d'un support des vêtements de travail (14a ; 14b ; 14c), dans lequel la grandeur caractéristique spécifique à l'utilisateur peut être détectée par au moins une unité de capteurs (62a ; 62b ; 62c) des vêtements de travail (14a ; 14b ; 14c).

10. Système d'entreposage selon l'une quelconque des revendications précédentes, **caractérisé par** au moins un dispositif de séchage et/ou de nettoyage (66a ; 66b ; 66c), qui est prévu pour effectuer une opération de séchage et/ou de nettoyage des vêtements de travail (14a ; 14b ; 14c) en fonction d'au moins une grandeur caractéristique détectée au moyen du dispositif de capteurs (28a ; 28a ; 28c) et/ou en fonction de données électroniques échangées au moyen du dispositif de communication (60a ; 60b ; 60c).

11. Système d'entreposage selon l'une quelconque des revendications précédentes, **caractérisé par** au moins un dispositif de sortie (68a ; 68b ; 68c) pour l'émission d'informations concernant au moins un état des vêtements de travail (14a ; 14b ; 14c).

12. Système d'entreposage selon l'une quelconque des revendications précédentes, **caractérisé en ce qu'**au moins le dispositif d'entreposage (12b ; 12c) est mobile ou est intégré dans une unité mobile (90c).
